(19) Europäisches Patentamt
European Patent Office
Office européen des brevets

(11) EP 0 562 054 B1

(12) EUROPEAN PATENT SPECIFICATION

(45) Date of publication and mention of the grant of the patent:
**22.09.1999 Bulletin 1999/38**

(21) Application number: **92912237.2**

(22) Date of filing: **01.05.1992**

(51) Int Cl.[6]: **C07F 9/53**, C07F 7/30, C07F 7/00, C07F 15/00, C07F 15/06, C07F 1/00

(86) International application number:
**PCT/US92/03662**

(87) International publication number:
**WO 92/19573 (12.11.1992 Gazette 1992/28)**

# (54) TRANSITION METAL COMPLEX FOR USE AS A DIAGNOSTIC OR THERAPEUTIC PHARMACEUTICAL

ÜBERGANGSMETALLKOMPLEX ZUR VERWENDUNG ALS DIAGNOSTISCHES ODER THERAPEUTISCHES ARZNEIMITTEL

COMPLEX D' UN METAL DE TRANSITION UTILISE COMME PRODUIT PHARMACEUTIQUE A DES FINS THERAPEUTIQUES OU DIAGNOSTIQUES

(84) Designated Contracting States:
**AT BE CH DE DK ES FR GB GR IT LI LU MC NL SE**

(30) Priority: **01.05.1991 US 694142**

(43) Date of publication of application:
**29.09.1993 Bulletin 1993/39**

(73) Proprietor: **THE CURATORS OF THE UNIVERSITY OF MISSOURI**
**Columbia, MO 65211 (US)**

(72) Inventors:
- **KATTI, Kattesh, V.**
  **Columbia, MO 65203 (US)**
- **VOLKERT, Wynn, Arthur**
  **Columbia, MO 65202 (US)**
- **KETRING, Alan, Reed**
  **Columbia, MO 65203 (US)**

(74) Representative: **Wharton, Peter Robert**
**Urquhart-Dykes & Lord**
**Tower House**
**Merrion Way**
**Leeds LS2 8PA (GB)**

(56) References cited:
- **CHEMICAL ABSTRACTS, vol. 93, no. 22, 1 December 1980, Columbus, Ohio, US; abstract no. 212784d, NISHIDA, Y. ET AL. 'ESR SPECTRA OF SEVEN-COORDINATED PENTAGONAL BIPYRAMIDAL COPPER(II) COMPLEXES' page 547 ; & INORG. CHIM. ACTA vol. 45, no. 3 , 1980 pages 113 - 114**
- **CHEMICAL ABSTRACTS, vol. 95, no. 8, 24 August 1981, Columbus, Ohio, US; abstract no. 72376m, NOETH, H. ET AL. 'STABILIZATION OF TRIS(HYDRAZINO)PHOSPHINE BY COMPLEX FORMATION' page 724 ; & Z. NATURFORSCH., B: ANORG. CHEM., ORG. CHEM. vol. 36B, no. 6 , 1981 pages 659 - 661**
- **CHEMICAL ABSTRACTS, vol. 91, no. 12, 17 September 1979, Columbus, Ohio, US; abstract no. 101291e, MAISCH, H. 'STABILIZATION OF PHOSPHANES P(NHX)3 BY LIGAND REACTIONS OF PENTACARBON YL(TRICHLOROPHOSPHANE)MOLYBDENUM(0 )' page 670 ; & Z. NATURFORSCH., B: ANORG. CHEM., ORG. CHEM. vol. 34B, no. 6 , 1979 pages 784 - 789**
- **Tetrahechon, Vol. 32, 1976, MAJORAL et al., "Keterocycler Content Du Phosphore XXXIX", pp. 2633-2644**
- **CHEMICAL ABSTRACTS, Volume 87, No. 19, issued 7 November 1977 (Columbus, Ohio, USA), GRAPOV et al., "Some reactions of phosphours, his acid dihydrazides", Abstract No. 152323s; & Zh obshch Khim (1977), 47(8), pp. 1704-11.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 562 054 B1

- CHEMICAL ABSTRACTS, Volume 88, No. 7, issued 13 February 1978 (Columbus, Ohio, USA), MAJORAL et al., "Heterocycles containing phosphous XXXII", Abstract No. 50967h; & Heterocyclic Chemistry (1977), 14(5), pp. 749-55.
- CHEMICAL ABSTRACTS, Volume 76, No. 20, issued 15 May 1972, HUANG et al., "Polymers containing phosphous", Abstract No. 113579h; & Kozyo Kogaher Zasshi (1971), 77(11), pp. 2406-9.
- Chemische Berichte, Vol. 100, No. 7 (1967), HORN et al., "Darstellung von Thiophosphoyltrihydrazide, ect.", pp. 2258-2260
- CHEMICAL ABSTRACTS, Volume 69, No. 25, issued 16 December 1968, SCOLER et al., "N,N'-Disubstituted P-phenylphosphonothioic diamcles", Abstract No. 106808; & J. Chem. Eng. Data (1968), 13(4), pp. 571-2.
- SCOLA et al., "N,N'-Disubstituted P-phenylphosphonothioic diamides", J. Chem. Eng. Data (1968), 13(4), pp. 571-572.

**Description**

RELATION TO OTHER APPLICATION

This application is a continuation-in-part of copending application Serial No. 694,142, filed May 1, 1991.

**MULTIFONCTIONAL LIGAND FOR USE AS A DIAGMOSTIC OR THERAPEUTIC PHARMACEUTICAL**

TECHNICAL FIELD

The present invention relates to pharmaceuticals, and especially radiopharmaceuticals for use as diagnostic and therapeutic agents. More specifically, the present invention relates to a compound and method of synthesizing a compound which is a multidentate ligand that complexes with transition metal radionuclides for use as diagnostic or therapeutic radiopharmaceuticals.

BACKGROUND OF THE INVENTION

Radiotherapy using "non-sealed sources" by way of radiolabeled pharmaceuticals has been employed for several decades (1-3).
Unfortunately, less than a handful of therapeutic radiopharmaceuticals are currently in routine use, as being approved by the FDA. There has been renewed interest in developing new agents due to the emergence of more sophisticated molecular carriers, such as monoclonal antibodies that are capable of selectively targeting cancerous lesions. In addition, the identification of several different radionuclides (4-7) with different chemical properties that have physical decay properties that are desirable for therapeutic application have further spurred development of new agents.
Despite some successes in treatment of specific malignant diseases and increased research and development activities in this area, many problems remain with the use of such treatments. For example, in most cancers, it has been difficult to provide acceptable selectivity in radiation doses delivered to target tissues relative to normal tissues. Successful development of new therapeutic radiopharmaceuticals requires improved localization of these agents in target tissues and/or increasing rates of clearance from non-target tissues. In both of these cases, it is imperative that the therapeutic radionuclide remain firmly associated with the radioactive drug in vivo for extended periods of time. These periods of time can extend from a few hours up to several days, depending on the pharmacokinetics and physical half-life of the radionuclide. No single radionuclide can be appropriate in formulating therapeutic agents since different half-lives and the energy of emitted particles is required for different applications (4-7) thereby making it essential that radiopharmaceuticals with different radionuclides be available.
Therapeutic agents have been primarily labeled with beta-particle emitting radionuclides. Most of the promising radionuclides are produced in nuclear reactors, however, some are accelerator produced (4-7). Several different chelating structures have been employed to maintain the association of these beta emitters with the drug (8-12). Many of these structures are not sufficiently stable and most, if not all, do not provide appropriate routes or rates of clearance of radioactivity from non-target tissues (13-14). Accordingly, there is a delivery of high radiation doses to normal tissues and a reduction of the therapeutic ratio. This lowers the amount of radiation dose that can be safely delivered to a target tissue. Development of new radionuclide chelates that link the radioactive metal to the radiopharmaceutical is necessary. Further, new approaches must be taken in order to identify radio-labeling techniques that produce chelates that are highly stable in vivo but have improved clearance characteristics from normal tissues.
Bi-functional chelating agents have been used to form stable metal complexes that were designed to minimize in vivo release of the metallic radionuclide from the radiopharmaceutical. For example, diethyltriaminepentaacetic acid (DTPA) forms rather stable chelates with a variety of metals. However, coupling of this ligand to monoclonal antibodies by one of its five carboxyl groups resulted in unacceptable in vivo stability with a variety of radionuclides (15). Linking of this compound by a side group attached to one of the carbon atoms on an ethylene bridging group provides improved stability in vitro and in vivo. The stability characteristics of these compounds are not ideal resulting in poor clearance of activity from certain non-target organs are poor.
Chelating agents based on diamidodithiol and triamidomonothiol backbones are used for forming small and stable hydrophilic complexes with several beta-emitting transition metals. These were developed by Fritzberg and colleagues (10) for labeled monoclonal antibody products for diagnostic and therapeutic applications. These chelates provide improved clearance characteristics from the liver, however, kidney retention of activity when using Fab or $(Fab)_2$ fragments of monoclonal antibodies labeled with these radionuclide chelates is higher than desirable.
A macrocyclic tetramine-based chelating agent that also has four methylene carboxylate side atoms has been used to form a copper complex that has a high in vitro and in vivo stability when linked to monoclonal antibodies. This chelate was first described for monoclonal antibody bioconjugation by Meares and coworkers (11,12). The chelate is

rather large. Clearance of activity from non-target organs has not been shown to be more efficient than the chelates mentioned above.

Recently, a mono-hydrazide bifunctional chelating agent has been described that forms somewhat stable $^{99m}Tc$ complexes (16,17). This particular a ligand can be first attached to a protein and then binds to $^{99m}Tc$ as it is chelated with glucoheptonate in aqueous solutions at or near neutral pH (17). Other work with mono-hydrazide ligands with other $Tc^{V}$ complexes demonstrates the reaction of the monohydrazides at the axial position with $Tc^{V}$=0 to form similar mondentate linkages (16). The in vitro and in vivo stability of these types of chelates has not been adequately described in the literature.

Applicant has synthesized a series of multi-dentate ligands derived from a phosphorous or germanium core utilizing hydrazine groups as arms of these ligands to form small but stable and well defined complexes with transition metal radionuclides. Unlike prior art chelates, these chelates show good stability in both aqueous solutions, serum, and other body fluids.

SUMMARY OF THE INVENTION

In accordance with the present invention, there is provided a compound for use as a diagnostic or therapeutic pharmaceutical, or MRI contrast agent, said compound comprising:

a phosphine core, and at least two hydrazine groups forming a ligand bound to a transition metal extending from said phosphorous core wherein said liaand is of the formula

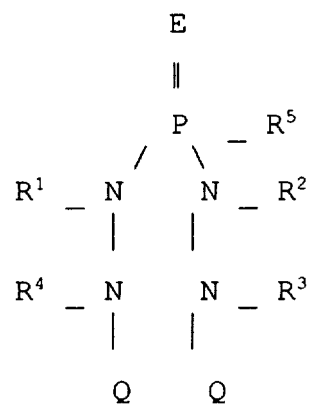

wherein $R^1$, $R^2$, $R^3$, $R^4$ are all the same or different and are H,

an alkyl group selected from Me, Et, - or i-propyl, n-, i- or t-butyl, n-hexyl, $-CH_2COOH$, $-CH(OH)CH_2OH)$, cyclohexyl,

an alkylamine group selected from $-NMe_2$, NHMe, $(CH_2)_nNMe_2$, $(CH_2)_nNHMe$, - $(CH_2)_nNH_2$,

an alkoxy group selected from $-O(CH_2)_nCH_3$, $(CH_2)_nOCH_3$, - $(CH_2)_n$-COOH,- $(CH_2)_nSH$,

an aromatic group of the formula $-C_6H_4R$ wherein R = H, $NH_2$, COOH, OH, NCS, CHO, activated esters, acid anhydrides, -hydroxy succinamide, and $SiMe_3$;

each of $R^3$ and $R^4$ may be replaced by bond to Q;

$R^5$ is

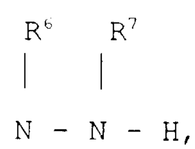

Me, Et, - or i-propyl, - i- or t- butyl, n - or cyclo - hexyl, primary or secondary alkyl amine, $-NMe_2$, $NHMe-(CH_2)_nNMe_2$, $-(CH_2)_nNHMe$, - $(CH_2)_nNH_2$, alkoxy $[O(CH_2)_{2n}(CH_3)_n]$, an aromatic group of the formula $C_6HySiMe_3$ or $-C_6H_4R^8$ wherein R $^8$ is H, $NH_2$, COOH, NCS, CHO, activated esters, acid anhydrides, N-hydroxysuccinimide, and SiMe $_3$; E is O, S, $NSiMe_3$, a lone pair of electrons, or $NC_6H_4R^8$; wherein $R^6$ and $R^7$ are the same or different from $R^1$, $R^2$, $R^3$ and $R^4$ but it is not selected from the moieties,$CH_2COOH$ and CHCOH/$CH_2OH$, $R^6$ can be any of the moieties defined for $R^5$ except for the hydrazine group

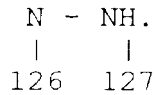

Q is H, $-CH_2Ph$ or -CHPh wherein in each instance Ph is unsubstituted or substituted with OH, COOH, O alkyl wherein alkyl are from 1 to 4 carbon atoms, $NH_2$, NH(alkyl $C_{1-4})_2$, or halogen such as chlorine, fluorine, bromine or iodine or Q can be $-CH_2$ piperazino or -CH piperazino; and wherein n is 1 to 6.

The present invention further provides a method of making a pharmaceutical comprising the transition complex of claim 1, said method including the following reaction:

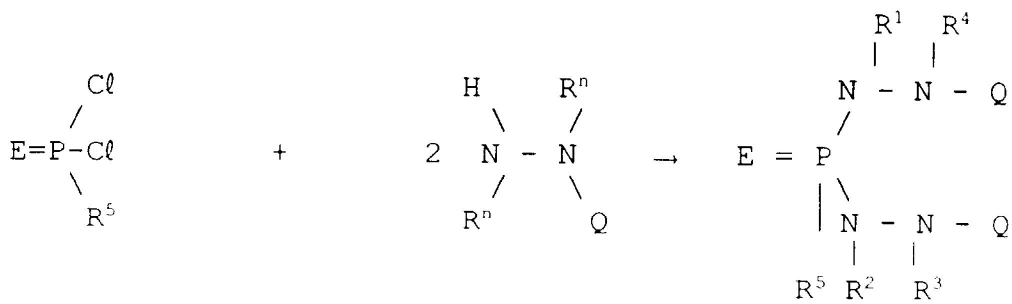

wherein $R^n$ represents $R^1$, $R^2$, $R^3$ or $R^4$ as defined in claim 1.

DETAILED DESCRIPTION OF THE INVENTION

Generally, the present invention provides a compound for use as a diagnostic or therapeutic pharmaceutical however, they may also be used for other pharmaceutical applications including MRI contrast agents. The compound includes a phosphine core and at least two hydrazine groups forming a ligand for bonding to a metal extending from the phosphorous or germanium core. That is, the invention provides phosphorous and germanium hydrazide ligand systems containing between 2 and 4 hydrazine units for use in forming complexes with a variety of transition metals that have high in vitro and/or in vivo stability.

The phosphorous and germanium ligands were chosen since the hydrazine arms linked directly to phosphorous atom provides a plethora of electron density on the terminal hydrazine-N-atoms that promote formation of highly stable nitrogen-metal bonds. This occurs even with the transition metals in their higher oxidation states, such as $Re^V$. The utilization of at least two hydrazine groups for metal bonding produces complexes that are more stable than the metal complexes with only one hydrazine arm.

The ligand is complexed with the transition metal, generally from the group including Fe, Mn, Re, Re, Pd, Rh, and $^{99m}Tc$. These complexes contain a 1:1 metal to ligand ratio which is formed making the resulting chelates small and well-defined. These specific combinations permit the formation of the complexes in a one step, high yield reaction as described below, especially for use with readily available chemical forms of the radionuclides. Other metals suitable for chelating are copper and cobalt.

For example, $^{99m}TcO_4^-$, $ReO_4^-$ chelates or $PdCl_2$ can be used. It has been determined that these types of ligands form highly stable chelates with a variety of transition metals that have radioactive isotopes that have potential for formulation of new therapeutic uses, such as $^{186}Re$, $^{188}Re$, $^{109}Pd$, $^{105}Rh$, etc., or for diagnostic use such as with $^{99m}Tc$ radiopharmaceuticals.

For example, Fe and Mn are paramagnetic elements and have potential application in chelate form as MRI

contrast imaging agents. Stable Mn and Fe chelates with the P and Ge hydrazine ligands with appropriate substituents can be formulated.

The chelates made in accordance with the present invention have been found to be stable in aqueous solutions, serum and other body fluids. This is critical to solve the problems of prior art agents which did not form stable chelates thereby having an inherent loss of control of localization of the radionuclide paramagnetic metal. Further, compounds made in accordance with the present invention can be chemically modified, as discussed below, to provide for specificity of localization, increased physical half-life of the radionuclide, improved pharmacokinetics, and increased selectivity of target tissues, such as tumors, over normal tissue, such as bone marrow, kidney, GI tract, liver etc.

The compounds made in accordance with the present invention are not only stable in neutral aqueous solutions, but have also been found to be stable in acidic and basic aqueous media. Again, this is critical with regard to localization of the compound in areas of the body having different pH's, as well as being stable through different administration routes, such as oral administration.

The above formulas characterize the present invention as being very modifiable in order to specifically tailor the ligand for chelation with a specific radionuclide and localization at a specific target organ.

For example, the ligand can conjugated to protein or antibodies and can use side chains previously used for linking monoclonal antibodies (15). For example, conjugation reactions ca involve reactive groups such as benzyl isothiocyanate, bromoacetamide, activated esters, N-hydroxysuccinimides, cleavable ester linkages, and aldehydes (15). In the case where E is a lone pair of electrons on the phosphorous atom, attachment of the chelate or the hydrazide ligand to proteins of other molecules already containing an azido group can be brought about by the standinger reaction. The conjugation reaction can occur using any of the R groups attached to the nitrogens on one of the hydrazine side arms or attached directly to the phosphorous or germanium atom. Accordingly, a single monoclonal antibody or several monoclonal antibodies can be added to the phosphorous or germanium hydrazine core to provide specificity of the binding of the ligand metal complex to specific surface antigen of target tissue.

As discussed above, other side chain modifications can be accomplished to make the chelate more polar and hydrophilic. For example, charged groups such as carboxyl or hydroxyl groups can be added at the various R groups appended to the hydrazine nitrogens. This additional small change in the compounds providing charged/polar groups increases the hydrophilic character of the resulting chelate. This will produce more rapid and selective clearance from the blood and nontarget tissue. This modification is highly desirable for the promotion of efficient clearance of radioactivity from nontarget tissues, such as blood, liver, kidney, and spleen following catabolism of conjugated radiolabeled monoclonal antibodies that are presently used for therapy.

Alternatively, the hydrophobicity of the chelate can be varied incrementally by varying the alkyl chain length of the side chains appended to the hydrazine nitrogens. For example, the R groups or the hydrazine side arms can be derivatized with hydrogen, methyl, ethyl, n-, or -i-propyl or ni-, or t-butyl. This is desirable because with some chelates, particularly those labeled with $^{99m}$Tc, an increase in the hydrophobicity of the chelate plays a major role in targeting uptake in selective tissues, such as in brain, heart and lung. Addition of alkyl groups to the chelating backbone increases the lipid solubility of the chelate. If the resulting chelate is neutral, either brain, heart, or lung imaging agents can be developed (18). Similarly, if the overall chelate charge is +1 myocardial imaging agents can be developed (19). Modifying the hydrophobicity of chelates with beta-emitting radionuclides for therapy will also change the clearance and uptake properties in target and nontarget tissues.

An alternative to varying the alkyl chain length of the R groups appended to the nitrogens of the hydrazines is to add other hydrophobic functional groups, such as alkyl methoxy and alkyl methoxys to the R groups. The use of ether side chains instead of the alkyl side chains will increase lipophilicity but also improves the rate of clearance of the chelate from the blood and other non-target tissues. Other side chain modifications to increase hydryphilicity, such as the addition ester groups or amide groups, can also be accomplished.

Another alternative modification of the chelate is to modify the charge or basic metal chelate core. It is possible to make the chelate with zero charge to produce a neutral hydropholic chelate. For example, the bis-hydrazine ligand can be modified by the addition of a side chain other than hydrogen at the R groups attached to one of the hydrazine terminal nitrogens. Alternatively, negatively charged groups can be added, such as $(-CH_2)_n$-SH or $(-CH_2)_n$-COOH.

All of the aforementioned modifications demonstrate the flexibility of compounds made in accordance with the present invention and further the ability to modify these compounds to alter the binding, elimination, and absorption of the compounds in order to tailor the compounds for specific organ targeting, dosing, and metabolism.

A solution of the appropriate phosphorous halide (e.g. $P(o)Cl_3$, $P(S)Cl_3$, $PCl_3$; $RPCl_2$.

The following are examples of ligands and chelates formed in accordance with the present invention.

**EXAMPLE 1 Ligands Synthesized**

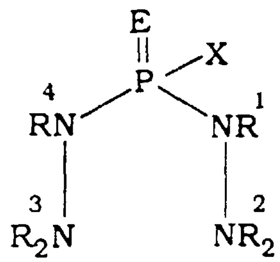

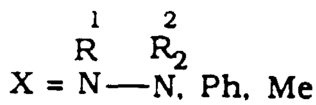

$R_1 = R_2 = R_3 = R_4 = H$; $R_1 = R_4 = Me$
E = O,S, $NSiMe_3$ or electron pair

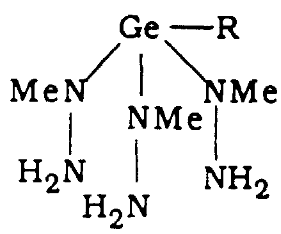

R = Cl; NMe—$NH_2$; Ph; Me

EXAMPLE II Formation of Complexes with Rhenium

1. Re-BHP Complexes formed:

a.

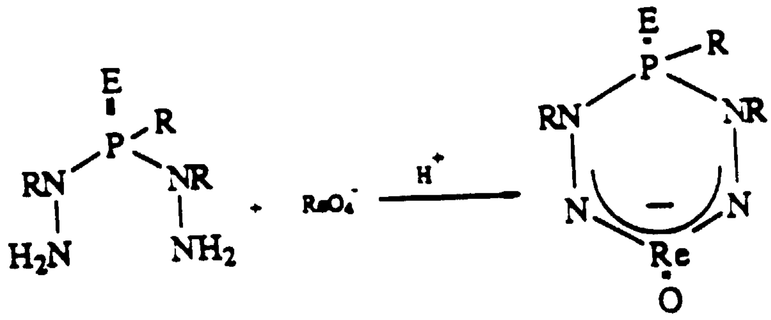


**Complex $I_{(Re)}$**

Characterized by $^1H$ and $^{31}P$ NMR and C,H,N elemental analysis

b. Cpd $II_{(Re)}$; $^1H$, $^{31}P$ NMR C,H,N El. anal.

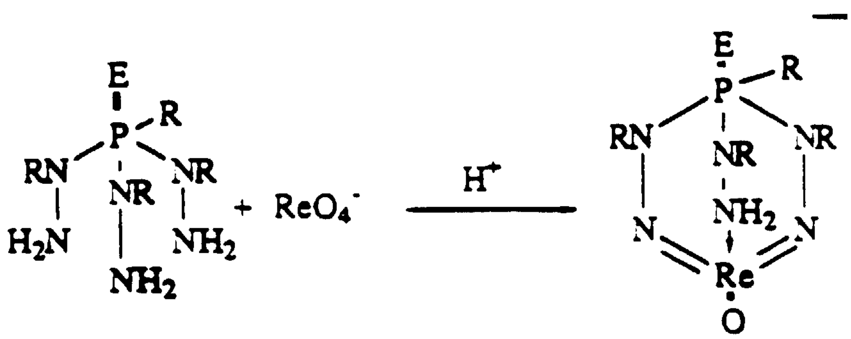


c. Formation of THP complexex with Re.

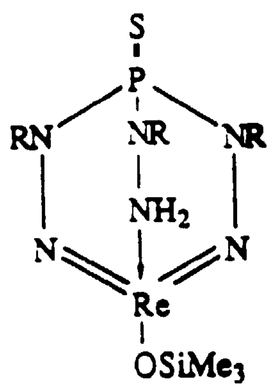

d. Formation of BHP complexes with Re

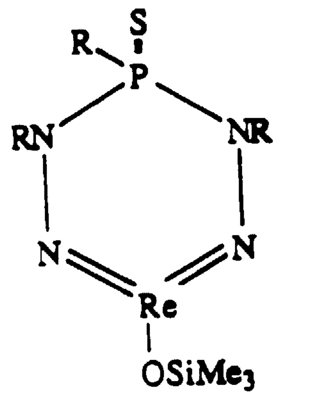

Cpd IV$_{(Re)}$, $^{1}H$, $^{31}P$ NMR C,H,N El. Anal

For the following examples unless otherwise stated, all reactions were carried out under anaerobic and anhydrous conditions using prepurified $N_2$ and conventional Schlenk techniques. Reagents such as $CO(C10_4)_2 6H_2O$, $Cu(ClO_4)_2 6H_2O$, $P(S)PhCl_2$ and $PdCl_2$ were purchased from Aldrich Chemical Co., USA and were used without further purification. Phenylphosphodihydrazide 1 was prepared by the reaction of $PhP(S)Cl_2$ with methyl hydrazine. (9,10).

Nuclear magnetic resonance spectra were recorded on a Bruker WH-500 Spectrometer. The $^{1}H$ NMR chemical shifts are reported in parts per million (ppm) downfield from external standard $SiMe_4$. The $^{31}P$ NMR spectra were recorded with 85% $H_3PO_4$ as an external standard and positive shifts lie downfield of the standard. The structure of the compounds prepared are set forth in Equation (1) and Schemes 2 to 4 hereof.

**Synthesis of Complex 2:** To a solution of 1(4.7 g; 20.25 mmol) in absolute ethanol (100mL) was added dropwise with stirring at 0°C a solution of $Co(ClO_4)_2 6H_2O$ (2.47g; 6.74 mmol) also in absolute ethanol (50mL). Upon completion of addition (30 minutes), a pink solid precipitated out. The mixture was stirred at 25°C for 6 hours before the solid precipitate was filtered and dried in air to obtain shiny light pink crystalline solid of analytically pure **2** (yield 3.93g; 95% based on $Co(ClO_4 6H_2O)$; mp 180°C dec. Anal. Calcd. for $C_{16}H_{30}N_8ClO_4P_2S_2CO$: C, 31.04; H, 4.85; N, 18.11; Cl, 573. Found: C, 31.02; H, 4.87; N, 18.10; Cl. 575.

**Synthesis of complex 3:** To a solution of 1 (4.53g: 19.70 mmol) in THF (150 mL) was added dropwise (30 minutes) with stirring at 0°C a solution of $Cu(clO_4)_2 6H_2O$ (1.82 g; 4.92 mmol) in absolute ethanol (50 mL). The mixture on stirring at 25°C for 8 hours turned greenish with suspensions of similar colored solid precipitate. The solvents were removed under vacuo and the crystalline residue was washed successively (4 x 25 mL) the THF to remove the unreacted excess of **1**. The leftover green solid was found to be analytically pure 3 (yield 2.80g; 91% based on Cu $(ClO_4)_2 6H_2O)$; mp 194C dec. Anal. Calcd for C, 30.81; H, 4.81; N, 17.97; Cl, 5.69. Found: C,30.84; H, 4.83; N, 17.96; Cl, 5.71.

**Synthesis of Complex 4:** A solution of $PdCl_2(PhCN)_2$ (3.53g; 9.22 mmol) in $CH_2Cl_2$ (100 mL) was added dropwise (15 minutes) at 25°C to a solution of 1 (2.12g; 9.22 mmol) also in $CH_2Cl_2$ (100 ml). The dark orange colored mixture on stirring for 4 hours turned yellow and the solvent was removed in vacuo to obtain a brown colored microcrystalline solid which upon washing with THF (2 x 10 ml) gave analytically pure **4** (yield 3.42g; 91%) mp 210°C dec. Anal. calcd. for $C_8H_{15}N_4Cl_2PSPd$: C, 23.56; H, 3.68; N, 13.74; cl, 17.40. Found: C,23.54; H, 3.70; N, 13.71; Cl, 17.51.

**Synthesis fo Compound 5**: To a solution of 1 (3.50g; 15.26 mmol) in absolute ethanol (100ml) was added dropwise (15 minutes) at 25°C with stirring a solution of salicyladehyde (3.81g; 31.28 mmol) in absolute ethanol (100 ml). The mixture was stirred under reflux for 12 hours before the solvent was removed under vacuo to obtain a white crystalline sold of **5** which was recrystallized from boiling acetonitrile (yield 6.4g; 96%); mp 80°C Anal. Calcd for $C_{22}H_{23}N_4O_2PS$: C, 60.22; H, 5.25; N, 12.78. Found C,60.21; H, 5.21; N, 12.74.

**Synthesis of Compound 6:** was synthesized by the reaction of **1** with piperazine aldehyde under identical reaciton conditions as described above for 5. Recrystallization from $CH_3CH/CHI_3$ (3:1) (yield 88%) mp 109°C. Anal. Calcd for $C_{18}H_{31}N_8PS$: C, 51.11; H, 7.34; N, 26.52. Found: C, 51.14; H, 7.37; N, 26.50.

**Synthesis of complex 7**: to a solution of **5** (2.95g; 6.73 mmol) in THF (100 mL) was added with stirring at 25°C a solution of $PdCl_2(PhCN)_2$ (2.58g; 6.73 mmol) also was removed under vacuo to obtain a brown microcrystalline solid of 7. The crude 7 was washed with chilled $CH_2Cl_2$(2 x 10 mL) to remove the residual benzonitrile before it was recrystallized from acetonitrile (yield 3.60g; 92%); mp 151°C (dec). Anal. Calcd for $C_{22}H_{22}N_4ClO_2PSPd$: C, 45.64; H, 3.83; N, 9.67; Cl, 6.12. Found: C, 45.57; H, 3.84; N, 9.70; Cl, 6.10.

**Synthesis of complex 8:** To a suspension of **6** (2.75g; 6.51 mmol) in dichloromethane (50 mL) was added

with stirring at 25°C a solution of $PdCl_2(PhCN)_2$ (2.49g; 6.51 mmol) also in dichloromethane (50 mL). The mixture was stirred for 6 hours before the solvent was removed in vacuo to obtain an orange colored microcrystalline solid of **8**. Recrystallization from boiling $CH_3CN$ gave analytically pure **8** (yield 3.25 g; 83%); mp. 163°C(dec). Anal. Calcd for $C_{18}H_{31}N_8Cl_2PSPd$: C, 36.02; H, 5.17; N, 18.67; Cl, 11.82. Found: C, 36.11; H, 5.15; N, 18.63; Cl, 11.84.

The invention has been described in an illustrative manner, and it is to be understood that the terminology which has been used is intended to be in the nature of words of description rather than of limitation.

Obviously many modifications and variations of the present invention are possible in light of the above teachings. It is, therefore, to be understood that within the scope of the appended claims, the invention may be practiced otherwise than as specifically described.

References

1 Spencer et al., *Radionuctides* in therapy, Boca Raton, FL: CRC Press, 1987.

2. Schlom, "Basis principles and applications of monoclonal antibodies in the management of carcinomas" *Canc Re*s 46:3225-3238, 1986.

3 Saenger et al., "Radiotherapeutic agents: properties, dosimetry and radiobiologic considerations" *Semin Nucl Med* 9:72-84, 1979.

4 Volkert et al., "Therapeutic Radionuclides: Production and decay property considerations" *J Nucl Med*32:1991

5 Schubiger and Hasler, *Radionuclides for therapy,* Basel, Switzerland: Hoffman-LaRoche & Co., Ltd., 1986.

6 Mausner et al., Production and use of prospective radionuclides for radioimmunotherapy. In: *Radiolabled monoclonal antibodies for imaging and therapy,* edited by Srivastava, S.C. New York. Plenum Publishing Corp., p. 149-163, 1988.

7. Andres et al., Radionuclides for therapy: a review In: *Radionuclides for Therapy,* edited by Schubiger, P. and Hasler, P. Basle, Switzerland: Editones-Roches, p. 9-20, 1986.

8 Kozak et al., "Nature of bifunctional chelating agent used for radioimmunotherapy with $^{90}$Y monoclonal antibodies. Critical factors in determining in vivo survival and organ toxicity" *Cancer Res* 39:2639-2644, 1989

9 Hnatowich, "Antibody radiolabeling, problems and promises" *Nucl Med Bio In J Radiat Appl Inst* [*B*] 17:49-55. 1990.

10 Rao et al., "Dependence of immunoreactivity and tumor uptake on ratio of Tc and Rc $N_2S_2$complexes per antibody $F_{ab}$ fragment" *J nucl Med* 29.815, 1988.

11 Deshpande et al., "$^{90}$Y-labeled monoclonal antibody for therapy; labeling by a new macrocyclic bifunctional chelating agent" *N Nucl Med* 31.473-479, 1990.

12 Washburn et al., "p-$NH_2$-Bz-DOTA-3A, a new bifunctional chelate reagent for labeling monoclonal antibodies with $^{90}$Y" *J Nucl Med* 31:824, 1990.

13 Meares et al., "Chelate radiochemistry: Clearable linkers lead to altered levels of radioactivity in the liver" *Int J Cancer* 2:99-102, 1988.

14 Naruki et al., "Differential cellular catabolism of $^{111}$In, $^{90}$Y and $^{125}$I radiolabeled T101 Anti-CD5 monoclonal antibody" *Nucl Med Biol; Int J Radiat Appl Inst* [*B*] 17:201-207, 1990.

15 Parker, "Tumour targeting with radiolabelled macrocycle-Antibody conjugates" *Chem Soc Rev* 19:271-291, 1990.

16 Abrams et al., "Synthesis and crystal and molecular structure of a Technetium-Hydralazino complex ..." *Inorg Chim Acta* 173:133-135, 1990*a*.

17 Abrams et al., "Technetium-99m-human polyclonal IgC radiolabeled via the Hydrazino Nicotinamide derivative for imaging focal sites of infection in rats" *J Nucl Med* 31:2022-2028, 1990*b*.

18 Kung et al., "Current and future radiopharmaceuticals for brain imaging with single photon emission computed tomography" *Sem Nucl Med* 20:290-302, 1990.

19 Dewanjee, "The chemistry of $^{99m}$Tc-labeled radiopharmaceuticals" *Sem Nucl Med* 20:5-27, 1990.

## Equation (1)

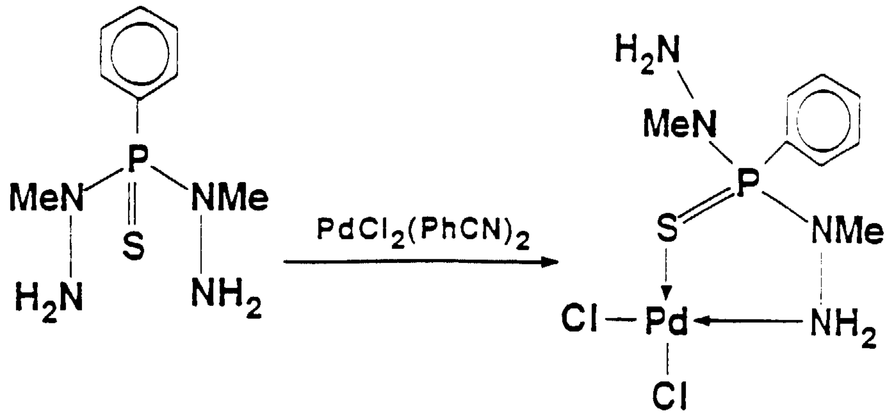


1 4

Scheme 2

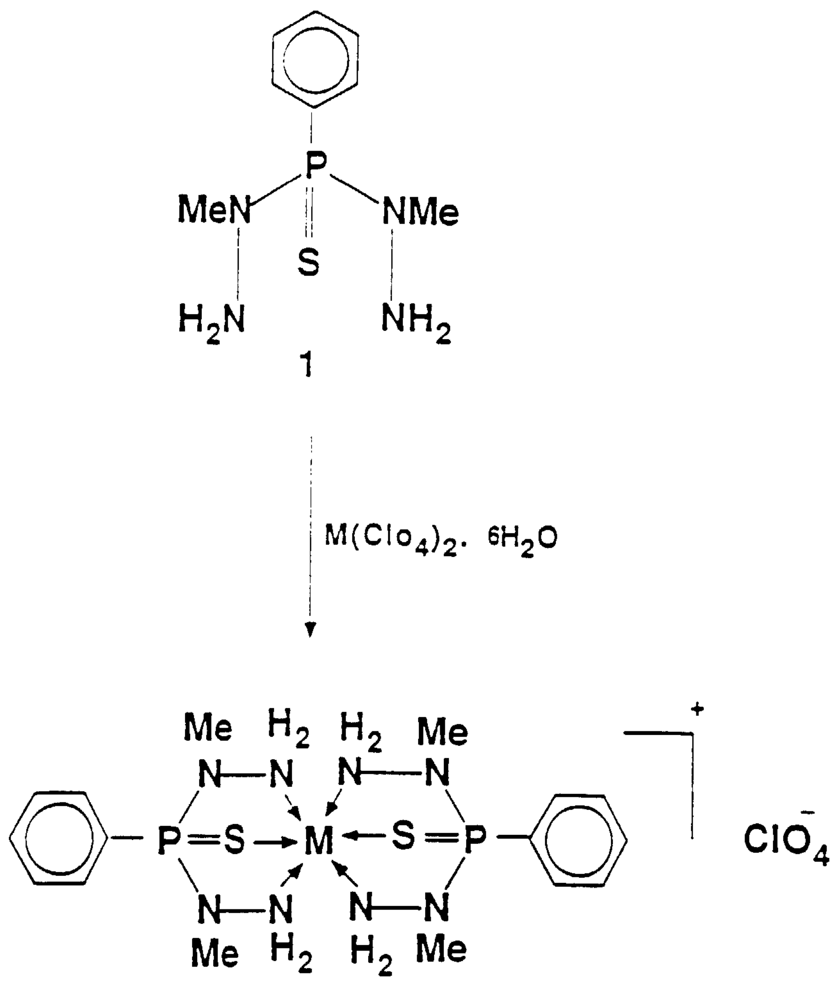


1

M = Co, 2 ; Cu,3

Scheme 3

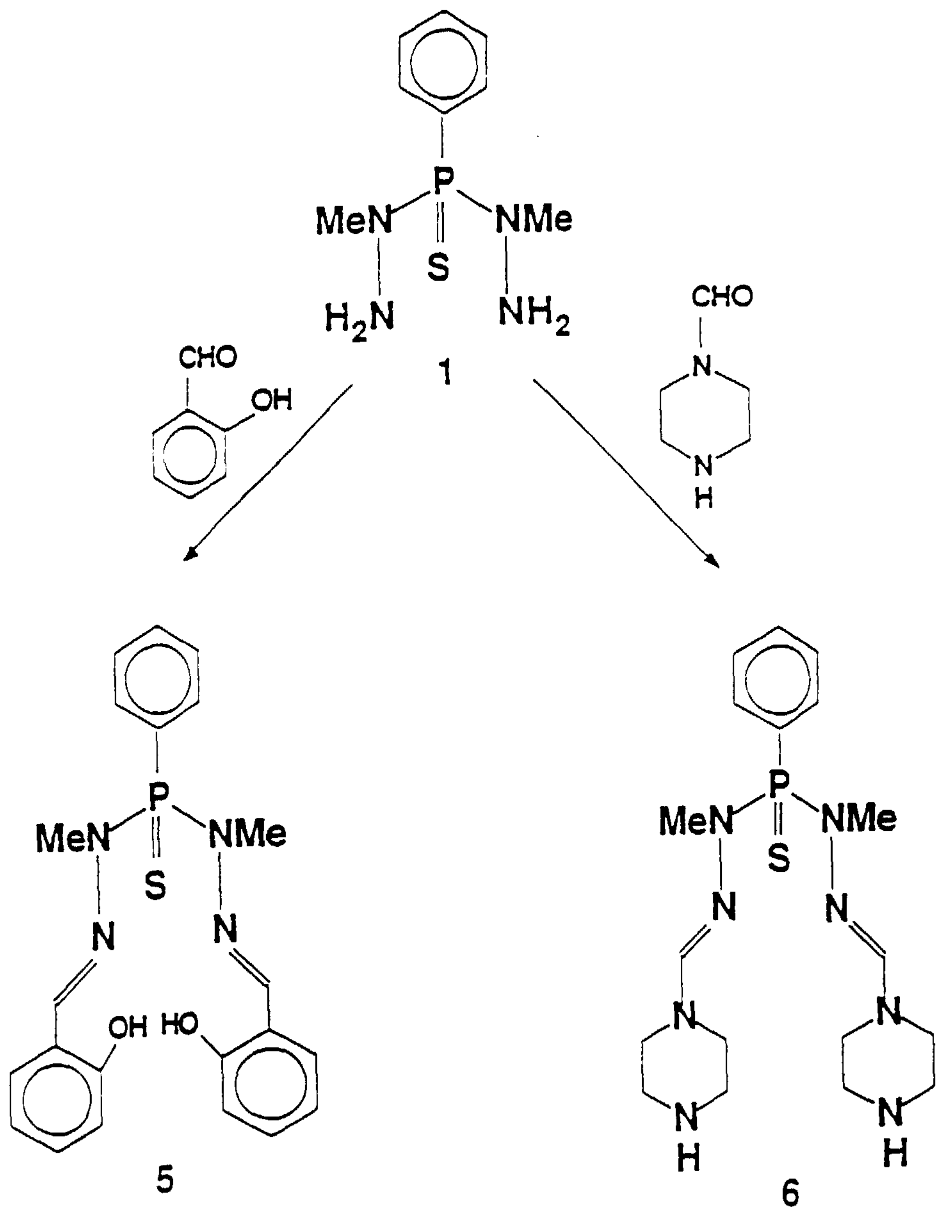

1

5

6

Scheme 4

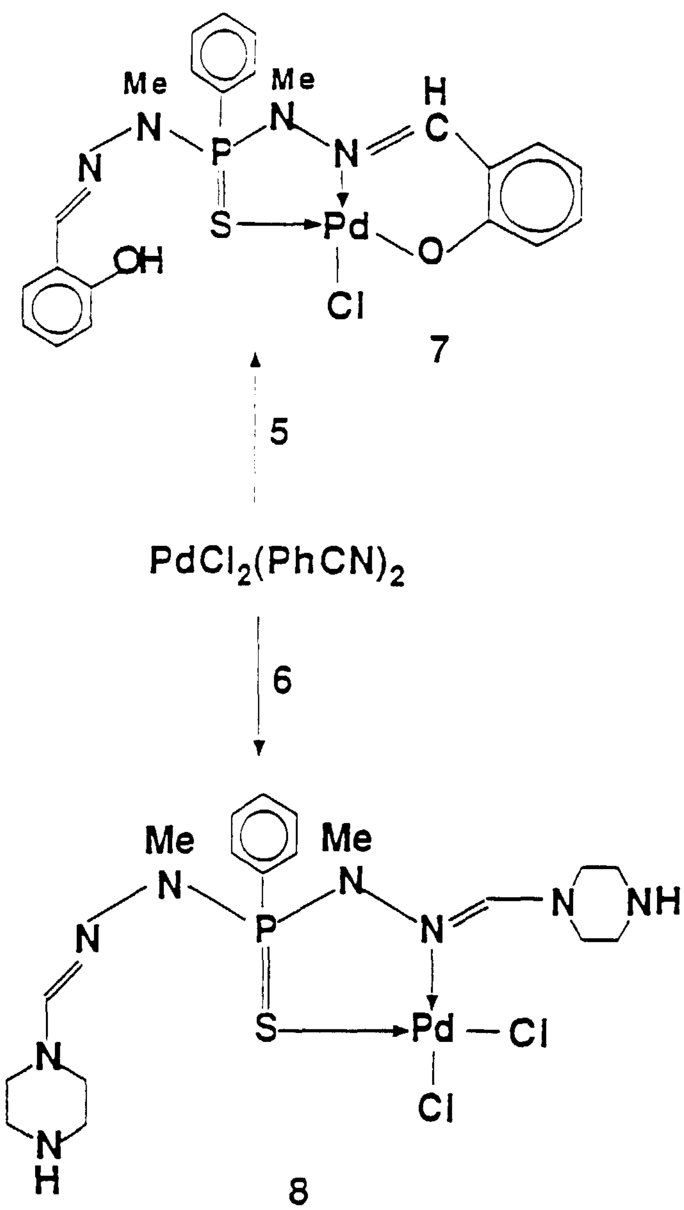


## Claims

1. A compound for use as a diagnostic or therapeutic pharmaceutical, or MRI contrast agent, said compound comprising:

   a phosphine core, and at least two hydrazine groups forming a ligand bound to a transition metal extending from said phosphorous core wherein said ligand is of the formula

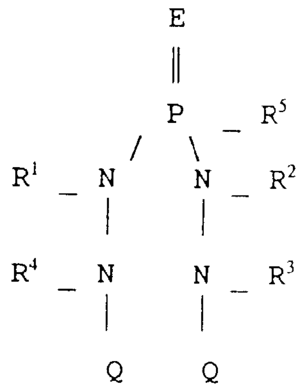

wherein $R^1$, $R^2$, $R^3$, $R^4$ are all the same or different and are H,

an alkyl group selected from Me, Et, n- or i-propyl, n-, i- or t-butyl, n-hexyl, $-CH_2COOH$, $-CH(OH)CH_2OH)$, cyclohexyl,

an alkylamine group selected from $-NMe_2$, NHMe, $(CH_2)_nNMe_2$, $(CH_2)_nNHMe$,$-(CH_2)_nNH_2$,

an alkoxy group selected from $-O(CH_2)_nCH_3$, $(CH_2)_nOCH_3$, - $(CH_2)_n$-COOH, $-(CH_2)_nSH$,

an aromatic group of the formula $-C_6H_4R$ wherein R = H, $NH_2$, COOH, OH, NCS, CHO, activated esters, acid anyhdrides, N-hydroxy Succinamide, and $SiMe_3$;

each of $R^3$ and $R^4$ may be replaced by a bond to Q $R^5$ is

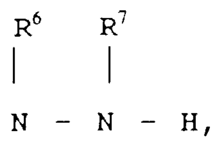

Me, Et, n- or i-propyl, n- i- or t- butyl, n - or cyclo - hexyl, primary or secondary alkyl amine,$-NMe_2$, NHMe-$(CH_2)_nNMe_2$, - $(CH_2)_nNHMe$, - $(CH_2)_nNH_2$, alkoxy $[O(CH_2)_{2n}(CH_3)_n]$, an aromatic group of the formula $C_6H_4SiMe_3$ or $-C_6H_4R^8$ wherein $R^8$ is H, $NH_2$, COOH, NCS, CHO, activated esters, acid anhydrides, N-hydroxysuccinimide, and SiMe $_3$; E is O, S, $NSiMe_3$, a lone pair of electrons, or $NC_6H_4R^8$; wherein $R^6$ and $R^7$ are the same or different from $R^1$, $R^2$, $R^3$ and $R^4$ but it is not selected from the moieties $CH_2COOH$ and CH $(OH)CH_2OH$, $R^6$ can be any of the moieties defined for $R^5$ except for the hydrazine group

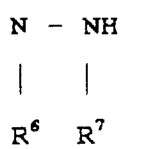 ;

Q is H, $-CH_2Ph$ or =CHPh wherein in each instance Ph is unsubstituted or substituted with OH, COOH, O alkyl wherein alkyl are from 1 to 4 carbon atoms, $NH_2$, $NH(alkyl\ C_{1-4})_2$, or halogen such as chlorine, fluorine, bromine or iodine or Q can be $-CH_2$ piperazino or -CH piperazino; and wherin n is 1 to 6

2. A compound according to claim 1 wherein said ligand is of the formula A:

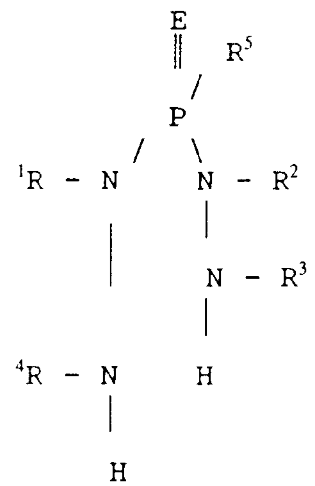

**3.** A compound as set forth in claim 1 wherein said ligand is a tris-hydrazine phosphine.

**4.** A compound as set forth in claim 3 wherein said ligand is of the formula:

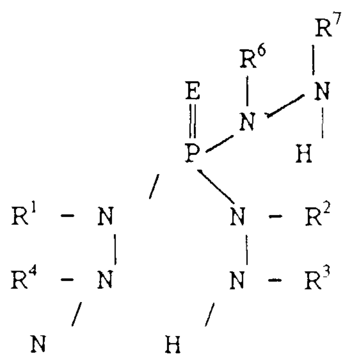

wherein $R^1$,$R^2$,$R^3$,$R^4$, and E have the meanings given in claim 1, $R^6$ and $R^7$ are selected from H,Me or Ph.

**5.** A compound as set forth in any of claims 1 to 4 wherein said transition metal is a radionuclide selected from the group including $^{186}Re$, $^{188}Re$, $^{109}Pd$, $^{105}Rh$ and $^{99m}Tc$, said compound being stable in aqueous solutions, serum and other body fluids.

**6.** A compound as set forth in claim 5 wherein said transition metal is a paramagnetic transition metal selected from the group includes Fe or Mn, said compound being stable in aqueous solutions, serum or other body fluids.

**7.** A compound as set forth in claims 5 or 6 including a 1:1 metal to ligand ratio.

**8.** A compound as set forth in any of claims 1 to 7 wherein said ligand is conjugated to a protein or antibody.

**9.** A compound as set forth in claim 8 wherein any one or several of $R^1$, $R^2$, $R^3$, $R^6$ or $R^7$ are benzyl isocyanate, bromacetamide, an ester, N-hydroxysuccinimide, or an aldehyde.

**10.** A compund as set forth in any of claims 1 to 7 wherein any one or all of $R^1$, $R^2$, $R^3$, $R^6$ or $R^7$ are carboxylated or hydroxylated to render said ligand more polar.

**11.** A method of making a pharmaceutical comprising the transition complex of claim 1, said method including the following reaction:

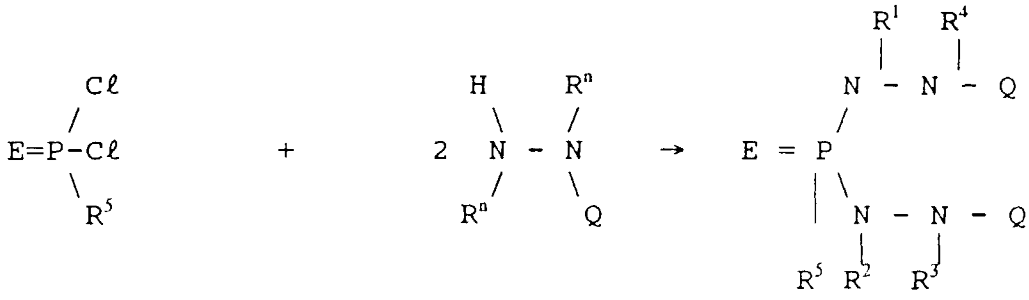

wherein $R^n$ represents $R^1$, $R^2$, $R^3$ or $R^4$ as defined in claim 1.

**12.** A method as set forth in claim 11 further including the step of chelating the compound to a transition metal selected from the group including Fe, Mn, $^{186}Re$, $^{188}Re$, $^{109}Pd$, $^{105}Rh$ and $^{99m}Tc$, said compound being stable in aqueous solutions, serum and other body fluids.

**13.** A method as set forth in claim 11 wherein the obtained ligand is conjugated to a protein or antibody.

**14.** A method as set forth in claim 13 further including the step of derivating $R^1$, $R^2$, $R^3$, $R^7$ or $R^6$, to a benzyl isocyanate, bromoacetamide, esters, N-hydryoxysuccinimide, aldehydes.

**15.** A method as set forth in claim 11 further including the step of modifying $R^1$, $R^2$, $R^3$, $R^7$ or $R^6$ to be more hydrophilic.

**16.** A method as set forth in claim 15 wherein said modifying step is further defined as hydroxylating or carboxylating $R^1$, $R^2$, $R^3$, $R^5$, or $R^6$.

## Patentansprüche

**1.** Chemische Verbindung zur Verwendung als pharmazeutisches Präparat für diagnostische oder therapeutische Zwecke oder als MRI-Kontrastmittel, welche folgendes aufweist:

einen Phosphinkern und mindestens zwei Hydrazin-Gruppen, die einen Liganden bilden, der an ein Übergangsmetall gebunden ist, das sich von dem phosphorhaltigen Kern aus erstreckt, wobei der Ligand der folgenden Formel entspricht:

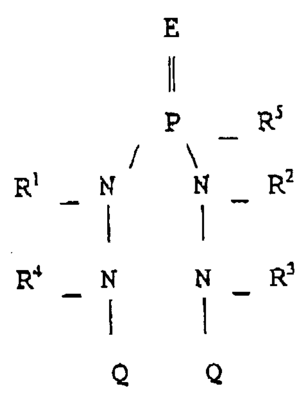

bei welcher $R^1$, $R^2$, $R^3$, $R^4$ alle gleich oder unterschiedlich sind und folgende sind: H,

eine Alkylgruppe, die aus Me, Et, n- oder i-Propyl, n-, i- oder t-Butyl, n-Hexyl, $-CH_2COOH$, $-CH(OH)CH_2OH)$, Cyclohexyl gewählt ist,

eine Alkylamin-Gruppe, die aus $-NMe_2$, NHMe, $(CH_2)_nNMe_2$, $(CH_2)_nNHMe$, $-(CH_2)_nNH_2$ gewählt ist,

eine Alkoxy-Gruppe, die aus $-O(CH_2)_nCH_3$, $(CH_2)_nOCH_3$, $-(CH_2)_n-COOH$, $(CH_2)_nSH$ gewählt ist,

eine aromatische Gruppe der Formel $-C_6H_4R$, wobei R = H, $NH_2$, COOH, OH, NCS, CHO, aktivierte Ester, Säureanhydride, N-Hydroxysuccinimid und $SiMe_3$ ist;

wobei $R^3$ und $R^4$ jeweils durch eine Bindung an Q ersetzt werden können;

wobei $R^5$ gleich

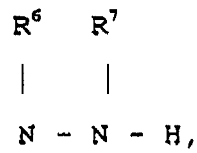

Me, Et, n- oder i-Propyl, n-, i- oder t-Butyl, n- oder Cyklohexyl, ein primäres oder sekundäres Alkylamin, $-NMe_2$, $NHMe-(CH_2)_nNMe_2$, $-(CH_2)_nNHMe$, $-(CH_2)_nNH_2$, Alkoxy$[O(CH_2)_{2n}(CH_3)_n]$, eine aromatische Gruppe der Formel $C_6H_4SiMe_3$ oder $-C_6H_4R^8$ ist,

wobei $R^8$ gleich H, $NH_2$, COOH, NCS, CHO, aktivierte Ester, Säureanhydride, N-Hydroxysuccinimid und $SiMe_3$ ist;

wobei E gleich 0, S, $NSiMe_3$, ein einzelnes Elektronenpaar oder $NC_6H_4R^8$ ist;

wobei $R^6$ und $R^7$ gleich $R^1$, $R^2$, $R^3$ und $R^4$ oder verschieden von diesen sind, jedoch nicht aus den Komponenten $CH_2COOH$ und $CH(OH)CH_2OH$ gewählt sind;

wobei $R^6$ jede der für $R^5$ definierten Komponenten sein kann, mit Ausnahme der Hydrazin-Gruppe

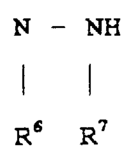 ;

wobei Q gleich H, $-CH_2Ph$ oder =CHPh ist, wobei in jedem Fall Ph nicht substituiert oder durch OH, COOH, O-Alkyl substituiert ist, wobei das Alkyl 1 bis 4 Kohlenstoffatome, $NH_2$, $NH(alkyl\ C_{1-4})_2$ oder ein Halogen ist, wie z. B. Chlor, Fluor, Brom oder Jod, oder Q gleich -CH2-Piperazin oder -CH-Piperazin sein kann; und n gleich 1 bis 6 ist.

**2.** Verbindung nach Anspruch 1,
bei welcher der Ligand der folgenden Formel A entspricht:

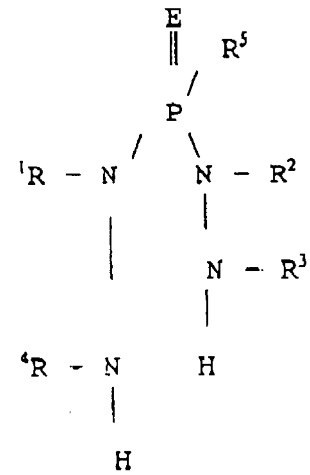

3. Verbindung nach Anspruch 1, bei welcher der Ligand Tris-Hydrazinphosphin ist.

4. Verbindung nach Anspruch 3, bei welcher der Ligand der folgenden Formel entspricht:

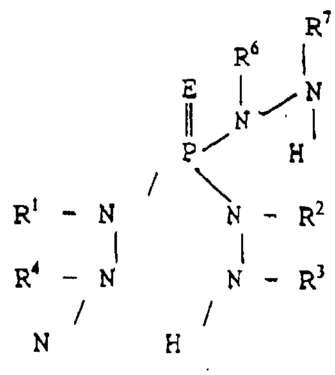

wobei $R^1$, $R^2$, $R^3$, $R^4$ und E die gleiche Bedeutung wie in Anspruch 1 haben und $R^6$ und $R^7$ aus der Gruppe H, Me oder Ph gewählt sind.

5. Verbindung nach einem der Ansprüche 1 bis 4, bei welcher das Übergangsmetall ein Radionuklid ist, das aus der Gruppe gewählt ist, welche $^{186}Re$, $^{188}Re$, $^{109}Pd$, $^{105}Rh$ und $^{99m}Tc$ umfaßt, wobei die Verbindung in wäßrigen Lösungen, Serum und anderen Körperflüssigkeiten stabil ist.

6. Verbindung nach Anspruch 5, bei welcher das Übergangsmetall ein paramagnetisches Übergangsmetall ist, das aus der Fe oder Mn enthaltenden Gruppe gewählt ist, wobei die Verbindung in wäßrigen Lösungen, Serum und anderen Körperflüssigkeiten stabil ist.

7. Verbindung nach Anspruch 5 oder 6, welche ein Verhältnis von 1:1 zwischen dem Metall und dem Liganden aufweist.

8. Verbindung nach einem der Ansprüche 1 bis 7, bei welcher der Ligand mit einem Protein oder einem Antikörper konjugiert ist.

**9.** Verbindung nach Anspruch 8, bei welcher eine oder mehrere der Gruppen $R^1$, $R^2$, $R^3$, $R^6$ oder $R^7$ Benzylisocyanat, Bromacetamid, ein Ester, N-Hydroxysuccinimid oder ein Aldehyd sind.

**10.** Verbindung nach einem der Ansprüche 1 bis 7, bei welcher eine oder alle der Gruppen $R^1$, $R^2$, $R^3$, $R^6$ oder $R^7$ carboxyliert oder hydroxyliert sind, um den Liganden stärker zu polarisieren.

**11.** Verfahren zur Herstellung eines pharmazeutischen Präparats, welches den Übergangskomplex nach Anspruch 1 aufweist, wobei das Verfahren die folgende Reaktion aufweist:

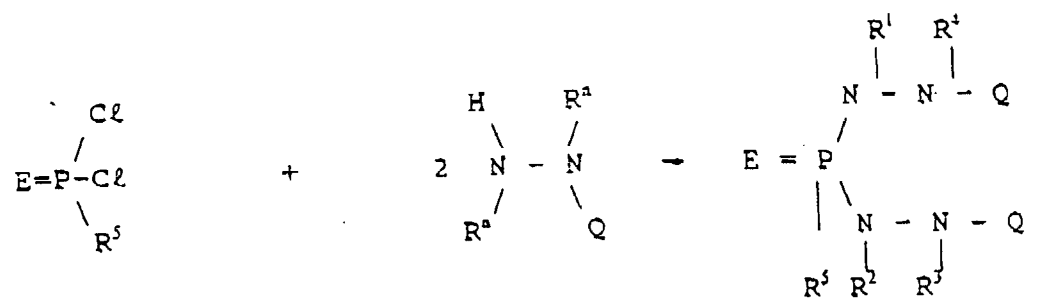

wobei $R^n$ die Gruppen $R^1$, $R^2$, $R^3$ oder $R^4$ gemäß der Definition in Anspruch 1 repräsentiert.

**12.** Verfahren nach Anspruch 11, welches den weiteren Schritt der Chelatbildung der Verbindung an einem Übergangsmetall aufweist, welches aus der Gruppe gewählt ist, die Fe, Mn, $^{186}Re$, $^{188}Re$, $^{109}Pd$, $^{105}Rh$ und $^{99m}Tc$ enthält, wobei die Verbindung in wäßrigen Lösungen, Serum und anderen Körperflüssigkeiten stabil ist.

**13.** Verfahren nach Anspruch 11, bei welchem die erhaltene Liganden-Verbindung an ein Protein oder einen Antikörper konjugiert ist.

**14.** Verfahren nach Anspruch 13, welches den weiteren Schritt der Derivatbildung von $R^1$, $R^2$, $R^3$, $R^7$ oder $R^6$ an einem Benzylisocyanat, Bromacetamid, Ester, N-Hydroxysuccinimid und Aldehyden aufweist.

**15.** Verfahren nach Anspruch 11, welches den weiteren Schritt der Modifizierung von $R^1$, $R^2$, $R^3$, $R^7$ oder $R^6$ aufweist, um diese stärker hydrophil zu machen.

**16.** Verfahren nach Anspruch 15, bei welchem der Modifizierungsschritt weiter als Schritt der Hydroxylierung oder Carboxylierung von $R^1$, $R^2$, $R^3$, $R^7$ oder $R^6$ definiert ist.

## Revendications

**1.** Composé destiné à être utilisé à titre de produit pharmaceutique à visée diagnostique ou thérapeutique, ou d'agent de contraste en RMN, ce composé comprenant :

un noyau de phosphine, et au moins deux groupes hydrazine formant un ligand lié à un métal de transition s'étendant depuis ledit noyau de phosphore, dans lequel ledit ligand a la formule suivante :

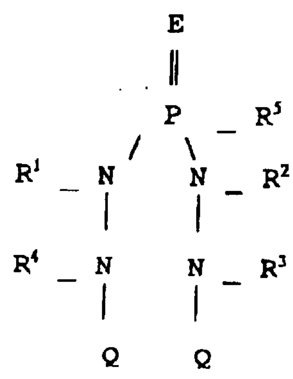

dans laquelle $R^1$, $R^2$, $R^3$, $R^4$ sont tous identiques ou différents et représentent H,
un groupe alkyle choisi parmi Me, Et, un n- ou i-propyle, un n-, i- ou t-butyle, un n-hexyle, $-CH_2COOH$, -CH (OH)$CH_2OH$, un cyclohexyle,
un groupe alkylamine choisi parmi $-NMe_2$, NHMe, $(CH_2)_nNMe_2$, $(CH_2)_nNHMe$, $-(CH_2)_nNH_2$,
un groupe alcoxy choisi parmi $-O(CH_2)_nCH_3$, $(CH_2)_nOCH_3$, $-(CH_2)_n-COOH$, $-(CH_2)_nSH$,
un groupe aromatique de formule $-C_6H_4R$, dans laquelle R = H, $NH_2$, COOH, OH, NCS, CHO, des esters activés, des anhydres acides, un N-hydroxysuccinamide, et $SiMe_3$ ;
chacun des $R^3$ et $R^4$ pouvant être remplacé par une liaison à Q $R^5$ représente

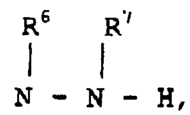

Me, Et, un n- ou i-propyle, un n-, i- ou t- butyle, un n- ou cyclo-hexyle, une alkylamine primaire ou secondaire, $-NMe_2$, NHMe-$(CH_2)_nNMe_2$, $-(CH_2)_nNHMe$, $-(CH_2)_nNH_2$, un alcoxy $[O(CH_2)_{2n}(CH_3)_n]$, un groupe aromatique de formule $C_6H_4SiMe_3$ ou $-C_6H_4R^8$, dans laquelle $R^8$ représente H, $NH_2$, COOH, NCS, CHO, des esters activés, des anhydrides acides, un N-hydroxysuccinimide, et $SiMe_3$ ; E représente O, S, $NSiMe_3$, une paire célibataire d'électrons, ou $NC_6H_4R^8$ ; et où $R^6$ et $R^7$ sont identiques ou différents de $R^1$, $R^2$, $R^3$ et $R^4$ mais ne sont pas choisis parmi les parties $CH_2COOH$ et CH (OH) $CH_2OH$, $R^6$ peut représenter l'une quelconque des parties définies pour $R^5$, excepté le groupe hydrazine

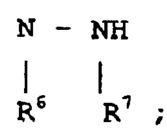

Q représente H, $-CH_2Ph$ ou =CHPh, où dans chaque cas, Ph est substitué ou non par OH, COOH, un O-alkyle, l'alkyle ayant de 1 à 4 atomes de carbone, $NH_2$, NH(alkyle $C_{1-4})_2$, ou un halogène tel que le chlore, le fluor, le brome ou l'iode ou Q peut être $-CH_2$ pipérazino ou -CH pipérazino ; et où n représente 1 à 6.

**2.** Composé selon la revendication 1, dans lequel ledit ligand a la formule A suivante :

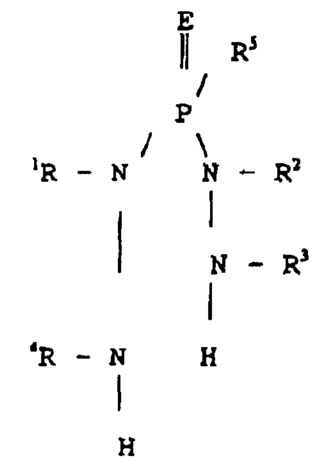

3. Composé selon la revendication 1, dans lequel ledit ligand est une tris-hydrazine-phosphine.

4. Composé selon la revendication 3, dans lequel ledit ligand a la formule suivante :

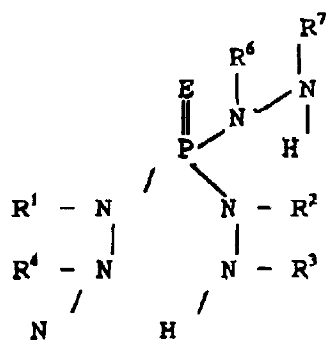

dans laquelle $R^1$, $R^2$, $R^3$, $R^4$ et E ont les significations données dans la revendication 1, $R^6$ et $R^7$ sont choisis parmi H, Me ou Ph.

5. Composé selon l'une quelconque des revendications 1 à 4, dans lequel ledit métal de transition est un radionucléide choisi dans le groupe comprenant $^{186}Re$, $^{188}Re$, $^{109}Pd$, $^{105}Rh$, et $^{99m}Tc$, ledit composé étant stable dans des solutions aqueuses, le sérum ou d'autres fluides corporels.

6. Composé selon la revendication 5, dans lequel ledit métal de transition est un métal de transition paramagnétique choisi dans le groupe constitué de Fe ou Mn, ledit composé étant stable dans des solutions aqueuses, le sérum ou d'autres fluides corporels.

7. Composé selon les revendications 5 ou 6 présentant un rapport métal à ligand de 1:1.

8. Composé selon l'une quelconque des revendications 1 à 7, dans lequel ledit ligand est conjugué à une protéine ou à un anticorps.

9. Composé selon la revendication 8, dans lequel un ou plusieurs des $R^1$, $R^2$, $R^3$, $R^6$ ou $R^7$ représentent l'isocyanate de benzyle, le bromacétamide, un ester, un N-hydroxysuccinimide, ou un aldéhyde.

10. Composé selon l'une quelconque des revendications 1 à 7, dans lequel un ou tous les $R^1$, $R^2$, $R^3$, $R^6$ ou $R^7$ sont carboxylés ou hydroxylés pour rendre ledit ligand plus 25 polaire.

11. Procédé de préparation d'un produit pharmaceutique comprenant le complexe de transition de la revendication 1,

ce procédé comprenant la réaction suivante :

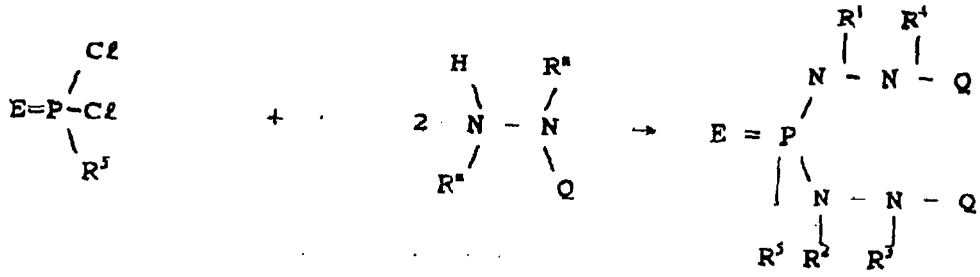

dans laquelle $R^n$ représente $R^1$, $R^2$, $R^3$ ou $R^4$, tels qu'ils sont définis dans la revendication 1.

**12.** Procédé selon la revendication 11 comprenant, en outre, l'étape de chélation du composé à un métal de transition choisi dans le groupe comprenant Fe, Me, $^{186}Re$, $^{188}Re$, $^{109}Pd$, $^{105}Rh$, et $^{99m}Tc$, ledit composé étant stable dans des solutions aqueuses, le sérum ou d'autres fluides corporels.

**13.** Procédé selon la revendication 11, dans lequel le composé obtenu est conjugué à une protéine ou à un anticorps.

**14.** Procédé selon la revendication 13 comprenant, en outre, l'étape de conversion de $R^1$, $R^2$, $R^3$, $R^7$ ou $R^6$ en isocyanate de benzyle, bromacétamide, esters, N-hydroxysuccinimide, aldéhydes.

**15.** Procédé selon la revendication 11 comprenant, en outre, l'étape de modification de $R^1$, $R^2$, $R^3$, $R^7$ ou $R^6$ pour les rendre plus hydrophiles.

**16.** Procédé selon la revendication 15, dans lequel ladite étape de modification est, en outre, définie comme une hydroxylation ou une carboxylation de $R^1$, $R^2$, $R^3$, $R^7$ ou $R^6$.